# EUROPEAN PATENT SPECIFICATION

(11) **EP 1 100 471 B1**
(45) Date of publication and mention of the grant of the patent: **17.09.2003**
(21) Application number: 99941487.3
(22) Date of filing: 28.07.1999
(51) Int. Cl.: A61K 9/20

(54) **COMPRESSED COMPOSITIONS COMPRISING CLARIFIED XANTHAN GUM**
KOMPRIMIERTE ZUSAMMENSETZUNGEN MIT GEKLÄRTER XANTHANGUMMI
COMPOSITIONS COMPRIMEES CONTENANT DE LA GOMME XANTHANE CLARIFIEE

(30) Priority: 01.08.1998 GB 9816723
(43) Date of publication of application: 23.05.2001
(73) Proprietor: The Boots Company PLC, Nottingham, Nottinghamshire NG2 3AA (GB)
(72) Inventor: DIXEY, Maureen, The Boots Company PLC, Nottingham, Nottinghamshire NG2 3AA (GB)
(74) Representative: Frith, Richard William
(86) International application number: EP9905499
(87) International publication number: WO00007569

(56) References cited:
- EP-A- 0 234 670
- EP-A- 0 306 454

## Description

This invention relates to controlled release formulations of therapeutic agents and in particular to sustained release formulations.

Sustained release formulations are employed where it is desired to administer a pharmacologically active ingredient to a patient over a prolonged period without requiring the patient to take repeated doses of the drug at short intervals. Such formulations may exhibit a range of release profiles. For example, the period over which drug is released may commence shortly after ingestion or, if the dosage form permits, the sustained release commences after a time. Some formulations may release one or more active ingredients at different rates. Some formulations are adapted to release the drug continuously until substantially all the active ingredient is released. Further formulations release the active continuously only for a particular period and then release the remaining active ingredient relatively quickly. Some formulations may have a linear, zero order or a first order release rate. Other formulations are adapted to have non-linear sustained release profiles. The desired release profile is generally determined by a number of factors including the nature of the active ingredient, the type of therapy and the nature of the excipient providing controlled release.

Various dosage forms may be used to provide sustained release. Most commonly, compressed dosage forms such as tablets are used. One method of achieving sustained release in tablets is to mix materials, such as polymeric materials, with the active ingredient and other necessary formulation ingredients and compress into a solid dosage form. An optional coating may be provided, which may itself have sustained release properties. The mixture comprising the sustained release carrier and active ingredient may be dispersed uniformly throughout the dosage form or it may form one or more layers in a multi-layered dosage form, such as a bi-layer tablet.

One polymer type which has been proposed for use as a sustained release agent is xanthan gum. This is especially advantageous as it can provide sustained release for a wide variety of active ingredients and is also capable of releasing the active ingredients over a very long period, eg up to 24 hours or more.

EP 306454A discloses that xanthan gum and/or pectin can be used as a bioadhesive agent in combination with a polyol to provide a composition having improved bioadhesion to mucous membranes and is capable of continual release of an active ingredient into the mouth of up to 3 hours. EP 234670A discloses that xanthan gum may be used in a compressed formulation, optionally in combination with other sustained release polymer materials, to provide sustained release of an active ingredient for up to 24 hours. WO 93/18758 also discloses a compacted sustained release composition for delivering a drug to the gastro-intestinal tract comprising an effective amount of the active ingredient admixed with xanthan gum. US 5419917 discloses that hydrogels such as hydroxymethylpropylcellulose, sodium alginate and xanthan gum may cause a drug to be released over a period of time with a zero-order release rate when the hydrogel is modified by the addition of an ionizable compound that is compatible with the hydrogel and affects the dissolution rate of the tabletted drug. WO 87/05212 discloses that polysaccharides of natural origin may be used to obtain retard matrices for the administration of active ingredients in solid dosage form. The retard matrix may comprise either xanthan gum alone or a mixture of xanthan gum with other natural or synthetic polymers. Among the polysaccharides of natural origin, modified corn starch, modified corn flour and xanthan gum are preferred. EP 360562A discloses a directly compressible free-flowing slow release granulation for use as a pharmaceutical excipient comprising about 20 to 70% by weight of a hydrophilic material comprising a heteropolysaccharide and a polysaccharide material capable of cross-linking the heteropolysaccharide in the presence of aqueous solutions and about 30-80% by weight of an inert pharmaceutical filler. This excipient can be mixed with a wide range of therapeutically active medicaments and then directly compressed into solid dosage forms. Preferably the heteropolysaccharide comprises xanthan gum or a derivative thereof and the polysaccharide material comprises one or more galactomannans.

Due to the structure of the gel formed *in vivo* xanthan gum is able to release active over prolonged periods, eg up to 24 hours or more. However, given that such a prolonged period of release is obtainable, it is important to ensure that dosage forms release the active ingredient in a profile that is substantially the same between different batches of the xanthan gum raw material.

In seeking to improve the reproducibility, many different variables can be considered, including, for example investigating the viscosity and rheology of the xanthan gum and other physical parameters such as pH, moisture content and particle size together with the chemical composition and impurities present in the xanthan gum; investigating the feedstock for the *Xanthomonas campestris* microorganism and the method by which the xanthan gum is produced and also collected and stored; considering the ingredients which may be used with xanthan gum in the formulation; investigating the degree and speed of compaction into tablets; examining the blending of xanthan gum with other sustained release polymers or swellable ingredients and/or investigating packaging and storage of the finished tablets.

We have now found that by using a particular type or form of the xanthan gum valuable release properties are obtained. The sustained release performance of the dosage form can significantly be improved in comparison with standard grades of xanthan gum in a way that was not predicted having regard to the prior art.

The present invention provides a solid sustained release pharmaceutical composition intended to release a pharmacologically active ingredient slowly after ingestion within the body as the composition progresses along the gastro-intestinal tract comprising a compressed mixture of said pharmacologically active ingredient and a sustained release carrier comprising xanthan gum characterised in that the composition comprises 10-25% by weight sustained release carrier and that the xanthan gum is capable of dissolving in water at a concentration of 1 part by weight xanthan gum to 100 parts by weight water to form a solution having a transmittance (600nm) of greater than 50%.

The light transmittance of the solution provides an indication of the clarity of the solution of xanthan gum in water. Preferably, the solution formed from the xanthan gum used in accordance with the present invention has a slightly cloudy or slightly milky appearance. More preferably, said solution has a substantially clear appearance, ie a 1% solution is substantially transparent on visual inspection. Its appearance may be contrasted with standard pharmaceutically acceptable grades of xanthan gum which have a significantly cloudy or turbid appearance when added to water. The cloudiness arises from the cell debris (resulting from the microbiological production process) which does not dissolve in water. The xanthan gum used in accordance with the present invention has reduced cell debris levels (preferably substantially no cell debris), accordingly, substantially all of the xanthan gum material dissolves to form a clarified solution compared to solutions of standard grades of xanthan gum, preferably a substantially clear solution. The type of xanthan gum material thus used in accordance with the present invention may be termed a clarified xanthan gum, preferably a transparent xanthan gum.

When measured by UV spectrophotometry at 600nm the light transmittance of a 1% w/w solution of said clarified xanthan gum in water was generally found to be in the range 60-100%, preferably 75-100%, more preferably 85-100% and most preferably 90-100%. This may be measured using a UV spectrophotometer, an apparatus and technique well known to those skilled in the art.

A particular advantage of the present invention is that a more reproducible release profile of active ingredient is obtained with the clarified (preferably transparent) grade of xanthan gum compared to other pharmaceutically acceptable grades of xanthan gum eg those having a transmittance of less than 10%. An improvement in the consistency in release profiles between formulations is important as it facilitates the manufacturing process ensuring that all the compositions fall within a desired specification. Furthermore, the production costs may be reduced through minimising the products which are wasted through falling outside the release profiles desired. In addition, the compositions are stable on storage.

The prior art relating to the use of xanthan gum in sustained release formulations has not suggested that a marked improvement in reproducibility can be obtained by using xanthan gum having a high transmittance value.

Xanthan gum is usually prepared by a microbiological process from the *Xanthomonas campestris* microorgansim. Methods for its production are well established in the art, see for example European Patent Application Numbers 78621, 68706, 66961, 66957, 66377, 28446 and US Patent Number 4352882. In an example process, xanthomonas campestris is cultured in a well-aerated medium containing glucose, a suitable nitrogen source, dipotassium hydrogen phosphate and trace elements. To provide seed for the final fermentation, the micro-organism is grown in several stages with associated identification tests prior to introduction into the final fermentation medium. At the conclusion of the fermentation process, xanthan gum is recovered by precipitation in isopropyl alcohol and is then dried and milled. The clarified type of xanthan gum used in accordance with the present invention is produced by techniques which reduce or remove the precipitated residue present at the last stage. This is carried out by established techniques well known to the person skilled in the art.

Preferably, the clarified xanthan gum has a viscosity greater than 400mPa, for example 400-2500mPa, more preferably 600-2000mPa, most preferably 1200-1600mPa (in 1% by weight salt solution).

Further preferably, when the clarified xanthan gum in accordance with the present invention is added to water the pH is in the range 5-9, more preferably 6-8.5 (1% by weight solution).

The mean particle size of the clarified xanthan gum used in the production process is preferably less than 1mm, more preferably less than 0.5mm, especially less than 0.25mm and most preferably less than 0.18mm.

The sustained release carrier is present to allow the release of the pharmacologically active ingredient from the composition over a period of time greater than that expected from a conventional immediate release tablet.

The composition comprises 10-25% by weight sustained release carrier. The sustained release carrier comprises clarified xanthan gum as defined herein. The sustained release carrier may also contain other ingredients which in combination with the xanthan gum contribute to the sustained release characteristics of the formulation. For example, ingredients which swell up in the presence of water contribute to the gel structure of the clarified xanthan gum. Examples of such materials include water-swellable polymers and other ingredients which absorb water. Thus, the sustained release carrier may contain a homogenous blended mixture of clarified xanthan gum with other water-swellable components. Preferably the sustained release carrier comprises 20% by weight or more of clarified xanthan gum, more preferably 40% or more of clarified xanthan gum and most preferably 50% or more of clarified xanthan gum, up to 100% by weight of clarified xanthan gum. The water swellable component may comprise one or more additional polymers having sustained release properties and which may be present to said clarified xanthan gum in a weight ratio 4:1, 3:1, 2:1, 1:1, 1:2, 1:3 or 1:4. We prefer to use not more than 50% by weight of the sustained release carrier preferably of such other sustained release polymers; thus the sustained release carrier comprises a major proportion of clarified xanthan gum. Examples of polymers having sustained release properties are water-swellable polymers eg cellulose ethers, locust bean gum, guar gum, carboxyvinyl polymer, agar, acacia gum, sodium alginate or alginic acid, or film-forming polymers eg ethyl cellulose, hydroxypropyl methylcellulose phthalate or acrylic resin. In a preferred formulation, the water-swellable component may comprise a water-swellable material normally associated with disintegrating properties when used in appropriate amounts, ie a water-swellable disintegrant. We prefer to use not more than 50% by weight of the sustained release carrier of water-swellable material, eg disintegrants. Examples of water-swellable materials include starch materials, such as pre-gelled starch, maize starch, potato starch, rice starch, tapioca starch, sodium and calcium carboxymethyl cellulose, silicon dioxide, croscarmellose sodium, soluble polyvinylpyrrolidone, magnesium aluminium silicate and modified starches such as sodium starch glycolate. Advantageous compositions according to the invention include a sustained release carrier comprising a major portion of said xanthan gum, preferably comprising 75-100% by weight of said clarified xanthan gum. More preferred compositions are those in which the sustained release carrier comprises 80-100% by weight (especially 90-100% by weight) of said clarified xanthan gum. Most preferably, the sustained release carrier consists essentially of said xanthan gum, for example 95-100% or even 98-100%. The improvement in reproducibility is obtained in comparison with a similar formulation containing xanthan gum having a transmittance of less than 50%.

The pharmacologically active ingredient may be any soluble or insoluble active ingredient suitable for use in sustained release formulations. Preferably, the active ingredient is insoluble or partially soluble. Representative types of orally active medicaments which may be incorporated in the sustained-release formulations according to the invention include, but are not limited to pharmacologically active ingredients to treat: the gastrointestinal tact (eg acid-peptic and mobility disorder agents, laxatives, antidiarrhoeals, colorectal agents and pancreatic agents for enzymes and bile acids); the cardiovascular system (eg arrhythmias and cardiac failure agents, antianginals, diuretics, antihypertensives, circulatory disorder agents, anticoagulants, antithrombotics, fibrinolytics, haemostatics, hypolipidaemic agents, anaemia and neutropenia agents); the central nervous system (eg hypnotics, anxiolytics, antipsychotics, antidepressants, anti-emetics, anticonvulsants, neurodegenerative disease agents and CNS stimulants); the endocrine system (eg male sexual disorder agents, corticosteroids, growth hormone and growth disorder agents, thyroid and antithyroid drugs, drugs affecting bone metabolism and vasopressin analogues); musculo-skeletal disorders (eg NSAIDs especially aryl propionic acid NSAIDs, disease modifying antirheumatic drugs, gout treatments, muscle relaxants, antiinflammatories, neuromuscular drugs); pain (eg analgesics, antipyretics and migraine treatments); diabetes (eg insulin, oral hypoglycaemic); infections and infestations (eg antibiotics, antibacterials, antifungals, antituberculous and antileptropic drugs, antimalarials, anthelmintics, amoebicides, antivirals, immunomodulators); the genito-urinary system (eg genital infection drugs, urinary tract infection drugs, renal and bladder disorder drugs); the respiratory system (eg bronchodilators, expectorants, antitussives, mucolytics and decongestants); vitamins, minerals and herbal remedies. A preferred group is especially aspirin and non-steroidal anti-inflammatory drugs (NSAIDs) in particular arylalkanoic acid, including their salts, esters, anhydrides, and other derivatives. These compounds are also antipyretics and analgesics. A further preferred group of active ingredients is the arylpropionic acids NSAIDs and salts thereof, including ibuprofen and flurbiprofen, their enantiomers and salts including S(+)-ibuprofen, S(+)-flurbiprofen, R(-)-flurbiprofen and the sodium or lysine salts thereof. More than one active ingredient may be used in the formulation. However, we prefer to use a single active ingredient.

In particular, when formulations comprise ibuprofen and a sustained release carrier according to the present invention, the formulations are therapeutically effective and exhibit valuable bioavailability characteristics. Furthermore, the sustained release observed when ibuprofen is the pharmacologically active ingredient may occur for as long as 24 hours, or even longer. Such a formulation provides a "once a day" formulation, thus allowing the patient to take only one dose, comprising one or more unit dosage forms, a day in order to achieve a therapeutically effective level of active ingredient.

The ratio of sustained release carrier according to the present invention comprising said clarified xanthan gum to pharmacologically active ingredient is preferably in the range 1:20 to 100:1.

For dosage forms containing a relatively high dose, in particular greater than 100mg of pharmacologically active ingredient, for example ibuprofen, then the ratio of the sustained release carrier of the present invention to pharmacologically active ingredient may be in the range 1:20 to 1:1, suitably 1:15 to 1:1 parts by weight. More preferred ratios fall within 1:10 to 1:1, and advantageously 1:5 to 1:2 parts by weight of the sustained release carrier to pharmacologically active ingredients.

For dosage forms containing a relatively low dose of pharmacologically active ingredient, ie less than 100mg and particularly less than 50mg, the above ratios may be reversed in order to provide a solid dosage form of a suitable size for administration to a patient, ie preferably within the range of ratios 20:1 to 1:1, suitably 15:1 to 1:1, especially 10:1 to 1:1, and advantageously 5:1 to 2:1 parts by weight of sustained release carrier to pharmacologically active ingredient. For very low dose pharmacologically active ingredients, ie particularly less than 10mg, the ratio of sustained release carrier to pharmacologically active ingredient may be in the range 100:1 to 1:1. preferably 50:1 to 1:1 parts by weight.

Preferred compositions according to the invention are obtained when the compositions comprise 65-90% by weight racemic ibuprofen or S(+)-ibuprofen and 10-35% by weight of a sustained release carrier comprising said clarified xanthan gum. Especially advantageous compositions comprise 80-90% by weight racemic ibuprofen or S(+)-ibuprofen and 10-20% by weight of a sustained release carrier comprising said clarified xanthan gum.

Advantageous formulations according to the invention comprise 10-50% by weight flurbiprofen, 10-30% by weight of a sustained release carrier comprising said clarified xanthan gum and 25-70% by weight pharmaceutically acceptable excipients, particularly 10-20% by weight flurbiprofen and 20-30% by weight of a sustained release carrier comprising said clarified xanthan gum together with 40-60% by weight of pharmaceutically acceptable excipients.

The sustained release medicament is provided in solid form, conveniently in a unit dosage form. The release profiles may be linear or non-linear depending on the nature of the active ingredient and the nature of the additional excipients used in the formulation. It may be formed into a sustained release medicament in a solid unit dosage form for oral administration, especially in tablet form. The tablet may release the drug over a period of time commencing shortly after ingestion. The tablet may optionally be provided with one or more layers which substantially prevent release until the dosage form reaches a certain point in the gastro-intestinal tract (eg determined by pH) or which act as a barrier and thus reduce the rate of release. There may also be provided optional layers which may release one or more active ingredients at different rates, eg substantially immediate release and sustained release or, particularly where two different active ingredients are employed, two different sustained release rates. The clarified xanthan gum defined in accordance with the present invention may be used in only one or may be used in each of the sustained release layers. The tablet is intended to release the pharmacologically active ingredient slowly after ingestion within the body as the formulation progresses along the gastro-intestinal tract In this regard, the gastro-intestinal tract is considered to be the abdominal portion of the alimentary canal, ie the lower end of the oesophagus, the stomach and the intestines.

Pharmaceutically acceptable excipients may also be incorporated into the sustained release formulation. Such pharmaceutically acceptable excipients may be added to modify the rate of drug dissolution and/or facilitate the manufacture of suitable dosage forms of the formulation.

For example, release-modifying pharmaceutically acceptable excipients that may be added in appropriate quantities for their particular ability to modify dissolution rates include, stearyl alcohol, hydrogenated cotton seed oil, polyethyleneglycol grades 4000 and 6000, surfactants such as sodium lauryl sulphate, polysorbates; lactose, sucrose, sodium chloride and tablet disintegrants for example corn starch, sodium starch glycolate, croscarmellose sodium and alginic acid. The quantity of such release-modifying excipients employed depend on the release characteristics required and the nature of the excipient. For a sustained release formulation according to the invention, the level of excipients used is suitably up to 25%, preferably up to 10% and advantageously up to 5% by weight of the total composition. Preferably the level of excipients is from 0.5-8% by weight, especially from 1-5% by weight.

Pharmaceutically acceptable excipients may also be incorporated into the sustained release formulation. Such pharmaceutically acceptable excipients may be added to modify the rate of drug dissolution and/or facilitate the manufacture of suitable dosage forms of the formulation.

For example, release-modifying pharmaceutically acceptable excipients that may be added in appropriate quantities for their particular ability to modify dissolution rates include, for example: stearic acid, metallic stearates, stearyl alcohol, hydrogenated cotton seed oil, polyethyleneglycol grades 4000 and 6000, surfactants such as sodium lauryl sulphate, polysorbates; lactose, sucrose, sodium chloride and tablet disintegrants for example corn starch, sodium starch glycolate, croscarmellose sodium and alginic acid. The quantity of such release-modifying excipients employed depend on the release characteristics required and the nature of the excipient. For a sustained release formulation according to the invention, the level of excipients used is suitably up to 25%, preferably up to 10% and advantageously up to 5% by weight of the total composition. Preferably the level of excipients is from 0.5-8% by weight, especially from 1-5% by weight.

The pharmaceutically acceptable excipients recognised by those skilled in the art, ie formulation excipients, which may be necessary for the formation of suitable dosage forms include, but are not limited to, binders for example polyvinylpyrrolidone, gelatin, pregelled starches, microcrystalline cellulose; diluents for example lactose, sodium chloride, dextrins, calcium phosphate, calcium sulphate; lubricants for example stearic acid, magnesium stearate, calcium stearate, Precirol (trade mark) and flow aids for example talc or colloidal silicon dioxide. If necessary, such formulation excipients may be used in large quantities particularly where the composition comprises a small amount of pharmacologically active ingredient. Preferably up to 50%, suitably up to 30% and especially up to 15% by weight of the composition of these above-mentioned excipients are employed.

The solid dosage form of the sustained release medicament may have an outer layer containing the same or a different active ingredient which is adapted to release the active ingredient quickly into the body. Optionally the dosage form is provided with a coating of any conventional coating material, eg a sugar or film coating material. If desired the coating material may have controlled release properties, eg an enteric coating and/or a sustained release coating optionally containing the same or a different active ingredient.

A sustained release formulation according to the invention may be formed into a solid dosage presentation according to conventional processes. The pharmacologically active ingredient and sustained release carrier comprising said clarified xanthan gum together with other optional pharmaceutically acceptable excipients are mixed and then compressed to produce a solid formulation. In one such method the pharmacologically active ingredient is mixed with a minor proportion of the sustained release carrier of the present invention to form a dry mixture of powders. The mixture is then granulated using a binder material in a solvent such as an alcoholic solvent, eg isopropyl alcohol or a mixture of a miscible organic solvent and an aqueous solvent. The wet granular mass is then dried. The other ingredients, including the remainder of the sustained release carrier of the present invention are dry mixed with the granules and compressed into tablets. Thus a minor proportion of said xanthan gum is included in a pre-granulated form of the active ingredient and a major proportion of said xanthan gum is combined with the pre-granulated active ingredient and basing excipients prior to compression into the solid dosage form. Alternatively, if the nature of the active ingredient permits, all the ingredients may be dry mixed. For example, a metoclopramide sustained release tablet may be produced by dry mixing together the pharmacologically active ingredient, sustained release carrier of the present invention and suitable pharmaceutically acceptable tabletting excipients to form a homogeneous blend, which is the compressed to give a tablet of the correct weight.

The solid formulations accosting to the invention should be compressed to a sufficient hardness to prevent the premature ingress of the aqueous medium into the core. In a preferred process, wherein a formulation according to the invention is processed into tablet form, advantageously the hardness of the tablets is of the order of 8-20kp as determined by a Schleuniger hardness tester.

Subject to the nature of the active ingredient, a formulation according to the invention is suitable for human or veterinary use.

The dosages of a formulation according to the invention correspond to the normal dosages of the particular active ingredient known to the person skilled in the art. The precise amount of drug administered to a patient will depend on a number of factors including the age of the patient, the severity of the condition and the past medical history, among other factors, and always lies within the sound discretion of the administering physician. For guidelines as to a suitable dosage, reference may be made to MIMS and to the Physicians Desk Reference.

As stated above, in a preferred pharmaceutical formulation according to the invention, the pharmacologically active ingredient is ibuprofen. Each dosage form suitably contains from 50 to 1200mg of ibuprofen, preferably from 200 to 800mg, and most preferably 300 to 600mg, in one or more unit dosage forms. The daily dosage as employed for an adult human treatment is generally in the range from 100 to 3200mg. Flurbiprofen is another pharmacologically active ingredient which may be used with advantage with a sustained release carrier according to the present invention comprising said clarified xanthan gum. Suitably the dosage of flurbiprofen is from 10-500mg per day. Suitably the unit dose compositions of the present invention contain 10-250mg, especially 6-100mg, preferably 12.5-50mg of the active ingredient. The daily dosage of the drug is generally in the range 10-500mg/day, more usually 30-300mg/day.

A particular advantage of the sustained release formulations of this invention is that high levels of ibuprofen and other suitable drugs can be employed. Thus, the present preferred compositions suitably comprise at least 50% by weight of ibuprofen, preferably at least 60-95%, especially from 75-90%.

In particular the provisions of a high dose composition having sustained release properties enables a unit dosage formulation of ibuprofen to be predicted which is suitable for once- or twice-a-day administration, preferably once-a-day.

The invention is illustrated by the following non-limitative Examples.

In the Examples the clarified or transparent xanthan gum used in accordance with the present invention and standard (non clear) grades (comparative Example) of xanthan gum are supplied under the trade names Keltrol T (transparent), Keltrol CR (clarified) and Keltrol standard by Monsanto Pharmaceutical Ingredients, Surrey, GB; colloidal silicon dioxide is available from Degussa, Frankfurt, DE under the trade name Aerosil 200; polyvinylpyrrolidone is available from ISP, Guildford, GB under the trade name Plasdone K29-32; carrageenan gum is supplied under the trade name Genuvisco; sodium alginate is supplied under the trade name Manugel by Monsanto Pharmaceutical Ingredients, Surrey, GB; microcrystalline cellulose is available from the FMC Corporation under the trade name Avicel PH101.

### Dissolution Test

The release rate was determined using the US Pharmacopoeia, 1995, Vol xxiii, Apparatus 2 modified by suspending the tablets in baskets 4 mm above the paddles, using a buffered aqueous medium at pH 8.6 (borate buffer) and with a paddle speed of 150 rpm.

Figure 1 illustrates the dissolution profiles of formulations according to the present invention. The % of ibuprofen dissolved over time is illustrated and shows the individual release rates of 8 batches of ibuprofen tablets prepared as described in Example 1.

Figure 2 illustrates the dissolution profiles over prior art formulations using a standard grade of xanthan gum.

The transmittance of the xanthan gum used in the Examples was measured by UV spectrophotometry at 600nm on a 1% w/w solution of the xanthan gum in water. The solution was prepared by dispersing the xanthan gum powder in purified water using a Silverson homogeniser at a speed in the range 2000-5000 rpm. The solutions were left overnight to allow de-aeration before analysis with a UV spectrophotometer. The % transmittance (%T) of different batches of each material compared with pure water (100% transmittance) is given below.

### A. % T of Xanthan Gum Used in Accordance with the Present Invention

| Test | Water %T | Xanthan Gum Grade | Xanthan Gum %T |
|---|---|---|---|
| 1 | 100.9 | Keltrol T | 96.3 |
| 2 | 100.0 | Keltrol T | 91.1 |
| 3 | 100.1 | Keltrol T | 91.3 |
| 4 | 100.0 | Keltrol CR | 68.2 |

### B. % T of Standard Grade Xanthan Gum Containing Production Residues (Comparative Example)

| Test | Water %T | Xanthan Gum Grade | Xanthan Gum %T |
|---|---|---|---|
| 1 | 100.0 | Keltrol Standard | 1.3 |
| 2 | 100.1 | Keltrol Standard | 0.7 |

### Example 1

Batches of sustained release tablets comprising 300mg ibuprofen were prepared from the following ingredients:-

| Ingredient | % w/w |
|---|---|
| Ibuprofen (25µm) | 77.2 |
| Transparent xanthan gum | 19.0 |
| Polyvinylpyrrolidone | 2.5 |
| Stearic acid | 1.0 |
| Aerosil | 0.3 |

Ibuprofen and approximately 20% of the total xanthan gum content were deaggregated through a 16 mesh screen into a blender and the dry powders mixed for two minutes at high speed. A solution of polyvinylpyrrolidone prepared in isopropyl alcohol was added to the mixing powder over a 30 second period. Further mixing and addition of isopropyl alcohol was carried out to produce suitable granules.

The wet granular mass was discharged through a 8 mesh screen into the drying bowl of a fluid bed dryer. The granules were dried until the moisture level reached below 1% w/w. The dry granules were forced through a 16 mesh screen, weighed and blended with the remainder of the xanthan gum and colloidal silicon dioxide for 20 minutes. The stearic acid was added and blended for a further four minutes. The blend was compressed on a tablet machine using pillow shaped tooling to produce tablets containing 300mg of ibuprofen.

The compressed cores were coated with a film coating comprising hydroxypropylmethylcellulose, pigment and French chalk.

In the same way, tablets comprising 200mg, 400mg, 600mg and 800mg ibuprofen can be prepared.

Furthermore tablets may be prepared (a) by including all of the Keltrol T in the granule and also (b) by including all of the Keltrol T in the basing ingredients (added after the granulation stage).

### Dissolution Results

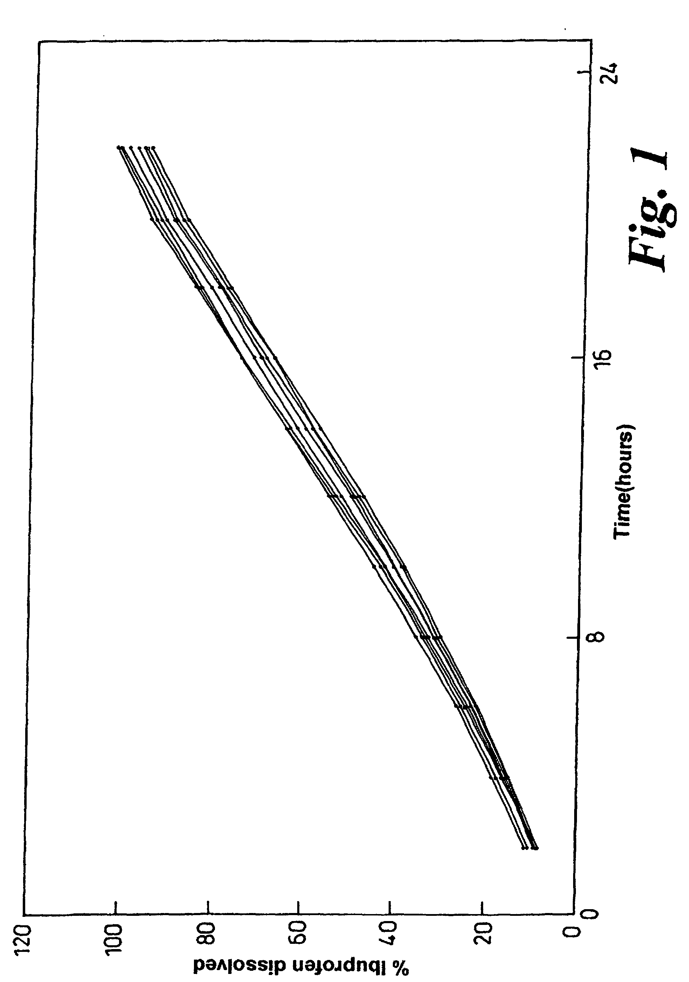

Eight different batches of Keltrol T (raw bulk material) were taken and six tablets prepared (as described above using laboratory scale compression machines) from each batch of raw material. The six tablets in each batch (A-H) were tested in the dissolution test described above to determine the percentage ibuprofen dissolved in the test solution over time. The mean value for the % of ibuprofen dissolved for the six tablets in each of the eight batches (A-H) are presented in Table 1(i) and (ii) below.
These results are also reproduced graphically in Figure 1.
It can be seen that
a) the tablets produce a regular rate of sustained release;
b) there is only a small variation in the percentage ibuprofen dissolved over a 22 hour release period, for example it can be seen that there is a variance of less than 10% being maintained throughout 22 hours for all eight batches of tablets. This reproducibility throughout the whole of this prolonged time period is particularly marked.

### Comparison with a Standard Grade of Keltrol

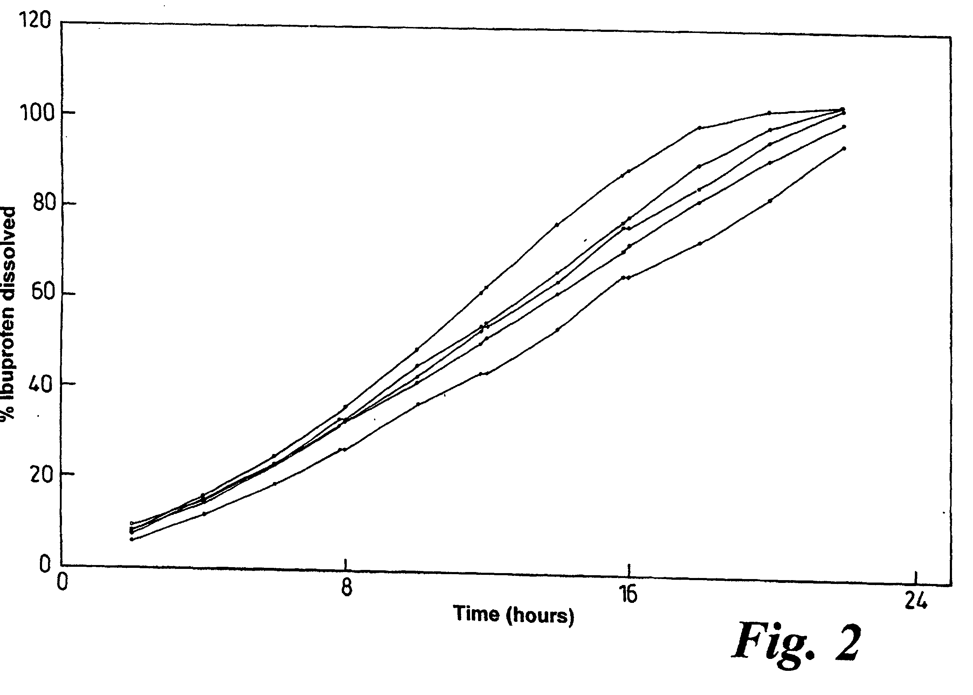

The results from Figure 1 (Keltrol T: transmittance >50%) can be contrasted with the results obtained with a standard grade of xanthan gum (Keltrol standard: transmittance <5%). Five different batches of standard Keltrol (as raw material) were taken, and six tablets prepared (as described above using laboratory scale compression machines) from each batch of raw material. The tablets were tested in the dissolution test described above. The mean value for the % of ibuprofen dissolved from the six tablets in each of the five batches (V-Z) are presented in Comparative Table 1 and also graphically in Figure 2. It can be seen that after 12 hours the amount of ibuprofen released can vary by 20% or more between the different batches of Keltrol (standard) raw material. It is noted that in the later stages of release the variation appears less marked as the % of ibuprofen released approaches 100%.

Accordingly, there is a substantial improvement in technical performance achieved by using the transparent grade of xanthan gum in contrast with the standard grade of xanthan gum.

**Table 1(i)**

| Time (hours) | % Ibuprofen Dissolved | | | |
|---|---|---|---|---|
| | Batch A | Batch B | Batch C | Batch D |
| 2 | 9.0 | 8.8 | 8.4 | 9.0 |
| 4 | 15.5 | 16.0 | 16.4 | 15.2 |
| 6 | 22.9 | 23.8 | 24.3 | 22.2 |
| 8 | 31.4 | 32.9 | 33.4 | 30.5 |
| 10 | 40.0 | 42.5 | 42.5 | 38.5 |
| 12 | 49.2 | 53.0 | 51.9 | 48.4 |
| 14 | 58.2 | 63.4 | 62.2 | 58.3 |
| 16 | 67.3 | 74.2 | 72.0 | 68.8 |
| 18 | 77.3 | 84.7 | 81.6 | 79.0 |
| 20 | 86.4 | 94.6 | 91.3 | 88.9 |
| 22 | 94.9 | 102.1 | 99.7 | 96.4 |

**Table 1(ii)**

| Time (hours) | % Ibuprofen Dissolved | | | |
|---|---|---|---|---|
| | Batch E | Batch F | Batch G | Batch H |
| 2 | 8.5 | 10.5 | 9.1 | 11.1 |
| 4 | 15.1 | 17.6 | 15.9 | 18.4 |
| 6 | 21.9 | 25.4 | 23.0 | 26.2 |
| 8 | 29.7 | 34.0 | 31.6 | 35.3 |
| 10 | 38.0 | 43.1 | 40.2 | 44.4 |
| 12 | 47.3 | 54.0 | 50.0 | 54.6 |
| 14 | 56.9 | 64.3 | 59.8 | 64.4 |
| 16 | 67.4 | 74.5 | 69.8 | 74.4 |
| 18 | 77.9 | 84.2 | 79.3 | 83.6 |
| 20 | 87.6 | 93.5 | 89.6 | 92.2 |
| 22 | 95.8 | 101.6 | 97.6 | 101.3 |

| Comparative Table 1 | | | | | |
|---|---|---|---|---|---|
| Time (Hours) | % Ibuprofen Dissolved | | | | |
| | Batch V | Batch W | Batch X | Batch Y | Batch Z |
| 2 | 7.6 | 9.4 | 8.4 | 6.1 | 8.3 |
| 4 | 15.4 | 14.9 | 15.9 | 11.8 | 14.3 |
| 6 | 23.1 | 22.6 | 24.5 | 18.4 | 22.6 |
| 8 | 32.2 | 32.1 | 35.8 | 26.0 | 32.8 |
| 10 | 41.2 | 42.6 | 49.0 | 36.3 | 45.0 |
| 12 | 51.5 | 54.7 | 63.1 | 43.6 | 54.0 |
| 14 | 61.5 | 66.2 | 77.1 | 53.6 | 64.2 |
| 16 | 72.4 | 78.4 | 89.5 | 65.6 | 76.2 |
| 18 | 82.2 | 90.4 | 99.2 | 73.2 | 85.2 |
| 20 | 91.4 | 98.7 | 102.6 | 83.0 | 95.6 |
| 22 | 99.7 | 103.4 | 103.6 | 95.1 | 102.6 |

### Example 2 : Flurbiprofen Tablet

Sustained release tablets containing 50 mg flurbiprofen were prepared from the following ingredients:-

| Ingredient | % w/w of tablet |
|---|---|
| Granule | |
| Flurbiprofen | 12.8 |
| Polyvinylpyrrolidone | 2.5 |
| Keltrol T | 4.0 |
| Microcrystalline cellulose | 37.8 |
| Dried maize starch | 18.0 |
| Croscarmellose sodium | 2.6 |

| Basing ingredients | |
|---|---|
| Stearic acid | 1.0 |
| Colloidal silicon dioxide | 0.3 |
| Keltrol T | 21.0 |

The flurbiprofen, microcrystalline cellulose, dried maize starch, croscarmellose sodium and Keltrol T were deaggregated through a 10 mesh screen into a blender and mixed to form a homogeneous blend. Granules were formed using a solution of the polyvinylpyrrolidone in isopropyl alcohol as the binding agent.

The wet granular mass was discharged through a 2 mesh screen and dried. The dried granules were passed through a 16 mesh sieve and combined with the colloidal silicon dioxide and the remainder of the Keltrol T. After thoroughly mixing, stearic acid was combined with the mixture. The blend was then compressed to produce tablets containing 50 mg flurbiprofen. The tablets were coated with a film coating comprising hydroxypropyl methylcellulose, pigment and French chalk.

In the same way, sustained release tablets containing 12.5 mg, 25 mg, 100 mg, 150 mg and 200 mg flurbiprofen (racemate) and S(+)-flurbiprofen can be prepared.

A batch of six tablets was tested as described in the dissolution test above and the results presented in Table 2.

**Table 2**

| Time (hours) | % Ibuprofen Dissolved | | | | | | |
|---|---|---|---|---|---|---|---|
| | A | B | C | D | E | F | Mean |
| 2 | 14.9 | 15.2 | 14.9 | 14.0 | 15.0 | 15.3 | 14.9 |
| 4 | 25.9 | 27.0 | 26.7 | 25.0 | 26.7 | 26.5 | 26.3 |
| 6 | 35.8 | 37.7 | 37.9 | 35.5 | 37.5 | 36.7 | 36.9 |
| 8 | 45.0 | 47.6 | 48.5 | 45.1 | 47.4 | 46.2 | 46.6 |
| 10 | 53.2 | 56.2 | 58.0 | 53.7 | 56.0 | 54.7 | 55.3 |
| 12 | 60.9 | 64.0 | 66.6 | 61.7 | 63.8 | 62.5 | 63.3 |
| 14 | 67.5 | 70.5 | 73.7 | 68.5 | 70.5 | 69.4 | 70.0 |
| 16 | 73.7 | 76.9 | 80.9 | 75.2 | 76.9 | 75.8 | 76.6 |
| 18 | 78.7 | 81.4 | 85.9 | 80.4 | 81.8 | 81.1 | 81.5 |
| 20 | 82.5 | 84.7 | 89.7 | 84.3 | 85.7 | 85.2 | 85.3 |
| 22 | 85.3 | 86.9 | 92.2 | 87.0 | 88.4 | 88.4 | 88.0 |

It can be seen that the six tablets produce a regular sustained release profile with only a small variance in the % of flurbiprofen released at each time point.

### Example 3: Sustained Release Flurbiprofen Tablets

Sustained release tablets containing 50 mg flurbiprofen were prepared from the following ingredients:-

| Ingredient | % w/w of tablet |
|---|---|
| Granule | |
| Flurbiprofen | 12.8 |
| Polyvinylpyrrolidone | 2.5 |
| Keltrol T | 4.0 |
| Microcrystalline cellulose | 58.3 |

| Basing ingredients | |
|---|---|
| Stearic acid | 1.0 |
| Colloidal silicon dioxide | 0.3 |
| Keltrol T | 21.0 |

In the same way as described in Example 2, granules of flurbiprofen were formed using a solution of the polyvinylpyrroidone in isopropyl alcohol as the binding agent, followed by combination with the basing ingredients and compression into tablets containing 50 mg flurbiprofen. The tablets were coated with a film coating comprising hydroxypropyl methylcellulose, pigment and French chalk.

in the same way sustained release tablets containing 12.5 mg, 25 mg, 100 mg, 150 mg and 200 mg flurbiprofen (racemate) and S(+)-flurbiprofen can be prepared.

A batch of six tablets was tested as described in the dissolution test above and the results presented in Table 3.

**Table 3**

| Time (hours) | % Ibuprofen. Dissolved | | | | | | |
|---|---|---|---|---|---|---|---|
| | A | B | C | D | E | F | Mean |
| 2 | 17.8 | 18.7 | 18.1 | 18.3 | 18.1 | 16.5 | 17.9 |
| 4 | 31.7 | 32.9 | 31.5 | 31.6 | 31.3 | 28.3 | 31.2 |
| 6 | 44.0 | 45.5 | 43.4 | 43.1 | 42.7 | 38.9 | 42.9 |
| 8 | 55.0 | 57.0 | 54.1 | 53.7 | 52.8 | 48.5 | 53.5 |
| 10 | 64.9 | 66.6 | 63.3 | 62.8 | 61.8 | 57.4 | 62.8 |
| 12 | 73.5 | 76.1 | 71.8 | 71.1 | 69.6 | 65.6 | 71.3 |
| 14 | 81.0 | 84.0 | 79.1 | 78.6 | 77.2 | 73.2 | 78.9 |
| 16 | 88.2 | 91.9 | 86.1 | 85.5 | 83.9 | 80.1 | 85.9 |
| 18 | 93.7 | 97.8 | 91.6 | 91.2 | 89.5 | 86.0 | 91.7 |
| 20 | 97.7 | 102.4 | 96.2 | 95.9 | 94.2 | 91.1 | 96.3 |
| 22 | 100.4 | 105.8 | 99.4 | 99.6 | 97.5 | 95.4 | 99.7 |

It can be seen that the six tablets produce a regular sustained release profile with only a small variance in the % of flurbiprofen released at each time point.

### Example 4: Ibuprofen Tablet

Sustained release tablets containing 335 mg ibuprofen were prepared from the following ingredients.

| Ingredient | % w/w of tablet |
|---|---|
| Granule | |
| Ibuprofen | 86.2 |
| Polyvinylpyrrolidone | 2.5 |
| Keltrol T | 1.6 |

| Basing ingredients | |
|---|---|
| Stearic acid | 1.0 |
| Colloidal silicon dioxide | 0.3 |
| Keltrol T | 8.4 |

In the same way as described in Example 1, granules of ibuprofen were formed using a solution of the polyvinylpyrrolidone in isopropyl alcohol as the binding agent followed by combination with the basing ingredients and compression into tablets.

In the same way, sustained release tablets containing 100 mg, 200 mg, 300 mg, 400 mg, 600 mg and 800 mg ibuprofen (racemate) and S(+)-ibuprofen can be prepared.

A batch of six tablets was tested as described in the dissolution test above and the results presented in Table 4.

**Table 4**

| Time (hours) | % Ibuprofen Dissolved | | | | | | |
|---|---|---|---|---|---|---|---|
| | A | B | C | D | E | F | Mean |
| 2 | 9.8 | 14.6 | 8.8 | 9.6 | 9.5 | 9.0 | 10.2 |
| 4 | 17.8 | 26.9 | 16.2 | 18.2 | 17.5 | 15.5 | 18.7 |
| 6 | 29.5 | 43.7 | 27.2 | 30.0 | 28.7 | 24.4 | 30.6 |
| 8 | 45.6 | 65.0 | 40.9 | 44.5 | 42.5 | 35.5 | 45.7 |
| 10 | 64.9 | 86.0 | 56.2 | 64.8 | 58.8 | 49.0 | 63.3 |
| 12 | 84.4 | 104.4 | 74.2 | 85.0 | 77.1 | 63.7 | 81.5 |
| 14 | 99.2 | 105.8 | 90.3 | 98.2 | 92.9 | 76.9 | 93.9 |
| 16 | 104.3 | 106.0 | 101.5 | 102.6 | 102.7 | 89.5 | 101.1 |
| 18 | 104.4 | 106.1 | 102.3 | 102.8 | 103.3 | 99.8 | 103.1 |
| 20 | 104.8 | 106.6 | 102.5 | 103.0 | 103.5 | 101.6 | 103.6 |
| 22 | 104.9 | 106.7 | 102.8 | 103.4 | 103.6 | 101.7 | 103.8 |

It can be seen that the six tablets produce a regular sustained release profile with only a small variance in the percentage of ibuprofen released at each time point.

### Example 5

Sustained release tablets containing 257 mg ibuprofen were prepared from the following ingredients.

| Ingredient | % w/w of tablet |
|---|---|
| Granule | |
| Ibuprofen | 66.2 |
| Polyvinylpyrrolidone | 2.5 |
| Keltrol T | 4.7 |

| Basing ingredients | |
|---|---|
| Stearic acid | 1.0 |
| Colloidal silicon dioxide | 0.3 |
| Keltrol T | 25.3 |

In the same way as described in Example 1, granules of ibuprofen were formed using a solution of the polyvinylpyrrolidone in isopropyl alcohol as the binding agent followed by combination with the basing ingredients and compression into tablets.

In the same way, sustained release tablets containing 100 mg, 200 mg, 300 mg, 400 mg, 600 mg and 800 mg ibuprofen (racemate) and S(+)-ibuprofen can be prepared.

A batch of six tablets was tested as described in the dissolution test above and the results presented in Table 5.

**Table 5**

| Time (hours) | % Ibuprofen Dissolved | | | | | | |
|---|---|---|---|---|---|---|---|
| | A | B | C | D | E | F | Mean |
| 2 | 6.6 | 6.6 | 6.9 | 6.8 | 6.6 | 6.6 | 6.7 |
| 4 | 12.1 | 12.0 | 12.6 | 12.2 | 12.0 | 12.0 | 12.2 |
| 6 | 17.5 | 17.5 | 18.0 | 17.7 | 17.5 | 17.4 | 17.6 |
| 8 | 22.7 | 23.1 | 23.6 | 23.1 | 23.0 | 22.7 | 23.0 |
| 10 | 28.0 | 28.6 | 28.9 | 28.3 | 28.6 | 28.2 | 28.4 |
| 12 | 33.2 | 34.3 | 34.6 | 33.7 | 34.4 | 33.9 | 34.0 |
| 14 | 38.7 | 40.1 | 40.4 | 39.4 | 40.6 | 40.0 | 39.9 |
| 16 | - | 45.8 | 46.6 | 45.1 | 46.8 | 45.7 | 46.0 |
| 18 | 49.0 | 51.4 | 52.7 | 50.6 | 53.1 | 51.3 | 51.4 |
| 20 | 53.6 | 57.1 | 58.7 | 56.1 | 59.2 | 57.0 | 57.0 |
| 22 | 59.6 | 62.6 | 64.7 | 61.7 | 65.0 | 62.5 | 62.7 |

The above test was terminated at 22 hours, although the tablet was still continuing to release ibuprofen.

### Example 6: Sustained Release Ibuprofen Tablet

Sustained release tablets containing 300 mg ibuprofen were prepared from the following ingredients.

| Ingredient | % w/w of tablet |
|---|---|
| Ibuprofen | 71.4 |
| Polyvinylpyrrolidone | 2.3 |
| Keltrol T | 18.8 |
| Sodium alginate | 6.2 |
| Stearic acid | 1.0 |
| Colloidal silicon dioxide | 0.3 |

The Keltrol T and sodium alginate formed the sustained release carrier and were blended by sieving through a 16 mesh sieve and then divided into two batches (batch A: 16% and batch B: 84%). The ibuprofen and batch A (sustained release carrier) was deaggregrated through a 6 mesh screen into a blender and mixed to form a homogenous blend. Granules were formed using a solution of the polyvinylpyrrolidone in isopropyl alcohol as the binding agent.

The wet granular mass was discharged through a 2 mesh screen and dried. The dried granules were passed through a 14 mesh sieve and combined with batch B (sustained release carrier). After thoroughly mixing stearic acid and colloidal silicon dioxide were combined with the mixture. The blend was then compressed to produce tablets containing 300 mg ibuprofen.

In the same way, sustained release tablets containing 100 mg, 200 mg, 400 mg, 600 mg and 800 mg ibuprofen (racemate) and S(+)-ibuprofen can be prepared.

A batch of six tablets was tested as described in the dissolution test above and the results represented in Table 6.

**Table 6**

| Time (hours) | % Ibuprofen Dissolved | | | | | | |
|---|---|---|---|---|---|---|---|
| | A | B | C | D | E | F | Mean |
| 2 | 10.8 | 12.9 | 10.6 | 11.6 | 10.7 | 10.7 | 11.2 |
| 4 | 22.0 | 29.6 | 20.5 | 25.1 | 21.2 | 20.8 | 23.2 |
| 6 | 56.9 | 95.9 | 35.8 | 94.1 | 52.2 | 37.2 | 62.0 |
| 8 | 95.9 | 96.9 | 90.2 | 96.1 | 96.7 | 97.2 | 95.5 |
| 10 | 96.1 | 97.0 | 90.3 | 96.0 | 96.8 | 97.4 | 95.6 |
| 12 | 96.3 | 97.3 | 90.4 | 96.1 | 97.0 | 97.4 | 95.8 |
| 14 | 96.6 | 97.6 | 90.5 | 96.2 | 97.2 | 97.5 | 95.9 |
| 16 | 96.8 | 97.6 | 90.6 | 96.5 | 97.4 | 97.7 | 96.1 |
| 18 | 96.9 | 97.9 | 90.7 | 96.4 | 97.4 | 97.8 | 96.2 |
| 20 | 97.3 | 98.0 | 90.9 | 96.6 | 97.6 | 98.1 | 96.4 |
| 22 | 97.3 | 98.3 | 90.8 | 96.7 | 97.8 | 98.0 | 96.5 |

It can be seen that the six tablets produce sustained release of the ibuprofen.

### Example 7: Chlorpheniramine Maleate Tablet

Sustained release tablets containing 12 mg chlorpheniramine maleate were prepared from the following ingredients:

| Ingredient | % w/w of tablet |
|---|---|
| Granule | |
| Chlorpheniramine | 3.1 |
| Polyvinylpyrrolidone | 2.5 |
| Keltrol T | 4.0 |
| Microcrystalline cellulose | 68.1 |

| Basing ingredients | |
|---|---|
| Stearic acid | 1.0 |
| Colloidal silicon dioxide | 0.3 |
| Keltrol T | 21.0 |

In the same way as described in Example 1, granules of chlorpheniramine maleate were formed using a solution of the polyvinylpyrrolidone in isopropyl alcohol as the binding agent, followed by combination with the basing ingredients and compression into tablets.

In the same way, sustained release tablets containing 4 mg, 8 mg, 16 mg, 20 mg and 24 mg chlorpheniramine can be prepared.

A batch of six tablets was tested as described in the dissolution test above and the results presented in Table 7.

**Table 7**

| Time (hours) | % Ibuprofen Dissolved | | | | | | |
|---|---|---|---|---|---|---|---|
| | A | B | C | D | E | F | Mean |
| 2 | 23.2 | 23.1 | 23.0 | 22.0 | 21.8 | 23.2 | 22.7 |
| 4 | 35.3 | 35.4 | 35.1 | 33.0 | 32.5 | 35.5 | 34.5 |
| 6 | 44.8 | 44.1 | 43.5 | 41.6 | 40.2 | 45.5 | 43.3 |
| 8 | 51.7 | 51.4 | 50.0 | 48.3 | 47.2 | 53.3 | 50.3 |
| 10 | 57.7 | 57.4 | 54.9 | 53.7 | 52.3 | 59.0 | 55.8 |
| 12 | 61.8 | 62.6 | 59.5 | 58.2 | 56.9 | 64.3 | 60.6 |
| 14 | 66.8 | 67.1 | 63.7 | 63.0 | 61.5 | 68.6 | 65.1 |
| 16 | 70.5 | 71.8 | 67.2 | 66.2 | 65.0 | 72.5 | 68.9 |
| 18 | 73.2 | 74.8 | 70.0 | 69.3 | 68.3 | 75.5 | 71.8 |
| 20 | 74.7 | 76.4 | 71.6 | 70.2 | 69.9 | 76.6 | 73.2 |
| 22 | 78.2 | 79.5 | 73.9 | 74.3 | 71.3 | 79.3 | 76.1 |

It can be seen that the six tablets produce a regular sustained release profile with only a small variance in the percentage of chlorpheniramine maleate released at each time point.

### Examples 8-28

Examples 8-10 and 13-25 may be made in the same way as described in Example 1 using the ingredients listed. Examples 11, 12 and 26-28 may be prepared by adding all the powdered ingredients to a mixer and blending to form a homogeneous mixture prior to compression into tablets. The proportion of clarified xanthan gum in the sustained release (SR) carrier is also given.

| Examples 8-16 | | | | | | | | | |
|---|---|---|---|---|---|---|---|---|---|
| **Ingredient** | **8** | **9** | **10** | **11** | **12** | **13** | **14** | **15** | **16** |
| Ibuprofen | 75.0 | 72.0 | 86.0 | - | - | 80.0 | 86.0 | 87.0 | 82.0 |
| Flurbiprofen | - | - | - | 35.7 | 35.7 | - | - | - | - |
| | | | | | | | | | |
| Keltrol T | 7.7 | 20.0 | 5.0 | 20.0 | 10.0 | 5.0 | 5.0 | 10.0 | 15.0 |
| Hydroxypropyl Cellulose | 9.5 | - | - | - | - | - | - | - | - |
| Carrageenan gum | 2.6 | - | - | - | - | - | - | - | - |
| Sodium alginate | | 5.0 | - | - | - | - | - | - | - |
| | | | | | | | | | |
| Lactose | 2.4 | - | 5.0 | 43.2 | 53.4 | 5.0 | 5.0 | - | - |
| Stearic acid | 1.0 | 1.0 | 3.0 | 1.0 | 0.90 | 3.0 | 3.0 | 1.0 | 1.0 |
| Polyvinylpyrrolidone | 1.90 | 2.0 | 1.0 | - | - | 1.0 | 1.0 | 2.0 | 2.0 |
| Silcon dioxide | - | - | - | - | - | 6.0 | - | - | - |
| | | | | | | | | | |
| **Clarified xanthan gum as % of SR carrier** | 39 | 80 | 100 | 100 | 100 | 100 | 100 | 100 | 100 |

| Examples 17-25 | | | | | | | | | |
|---|---|---|---|---|---|---|---|---|---|
| **Ingredient** | **17** | **18** | **19** | **20** | **21** | **22** | **23** | **24** | **25** |
| Ibuprofen | 84.5 | 80.0 | 84.0 | 83.0 | 82.0 | 77.0 | 80.0 | 72.0 | 71.0 |
| | | | | | | | | | |
| Keltrol T | 5.0 | 2.5 | 7.5 | 7.5 | 10.0 | 13.0 | 13.0 | 20.0 | 23.0 |
| Carrageenan gum | 2.5 | 7.5 | 2.5 | - | - | - | - | - | - |
| Sodium alginate | - | - | - | 7.5 | 5.0 | 5.0 | 5.0 | 5.0 | 3.0 |
| | | | | | | | | | |
| Lactose | 5.0 | - | 6.0 | - | - | - | - | - | - |
| Stearic acid | 1.0 | 3.0 | 1.0 | 1.0 | 1.0 | 1.0 | 1.0 | 1.0 | 1.0 |
| PolyvinylPyrrolidone | 2.0 | 1.0 | 1.0 | 2.0 | 2.0 | 2.0 | 2.0 | 2.0 | 2.0 |
| Silcon dioxide | - | 6.0 | - | - | - | 2.0 | - | - | - |
| | | | | | | | | | |
| **Clarified xanthan gum as % of SR carrier** | 67 | 25 | 75 | 50 | 67 | 72 | 72 | 80 | 88 |

| Examples 26-28 | | | |
|---|---|---|---|
| **Ingredient** | **26** | **27** | **28** |
| Metaclopramide | 12.3 | - | - |
| Indomethacin | - | 46.1 | - |
| Theophylline | - | - | 46.1 |
| | | | |
| Keltrol T | 28.0 | 23.0 | 28.0 |
| | | | |
| Lactose | 12.3 | 12.3 | 12.3 |
| Microcrystalline cellulose | 43.9 | 15.0 | 10.0 |
| | | | |
| Stearic acid | 1.0 | 1.0 | 1.0 |
| Polyvinylpyrrolidone | 2.5 | 2.5 | 2.5 |
| | | | |
| **Clarified xanthan gum as % of SR carrier** | 100 | 100 | 100 100 |

### Example 29: Dissolution Results on Storage

This improvement in reproducibility with clarified xanthan gum is also obtained on storage as shown in Tables 8, 9 and 10 below.
Three different batches of Keltrol T (as raw material) were taken and six tablets prepared (as described in Example 1 above using production scale compressing machines) from each batch of raw material. The tablets were then stored under certain conditions for four time periods as follows:-
(a) 3 months: 25°C and 60% relative humidity
(b) 3 months: 30°C and 60% relative humidity
(c) 3 months: 40°C and 75% relative humidity
(d) 6 months: 25°C and 60% relative humidity
(e) 6 months: 30°C and 60% relative humidity
(f) 6 months: 40°C and 75% relative humidity
(g) 9 months: 25°C and 60% relative humidity
(h) 9 months: 30°C and 60% relative humidity
(i) 12 months: 25°C and 60% relative humidity
(j) 12 months: 30°C and 60% relative humidity

After storage, the 3 batches (Batch I, J and K) of six tablets were tested in the dissolution test described above. The mean value of % ibuprofen dissolved for the six tablets in each test is presented below:-
Batch I:Tables 8(i) and (ii)
Batch J: Table 9(i) and (ii)
Bach K: Table 10 (i) and (ii)

From the Tables 8, 9 and 10 below, it can be seen that the advantageous reproducibility results are maintained on storage for extended periods at elevated temperature and a high relative humidity. It can be seen that the initial results obtained before storage are substantially the same as storage at 3 months at both 25°C and at 30°C. The extreme conditions encountered after storage at 40°C and 75% relative humidity have an effect on the gel and increase the dissolution rate of the ibuprofen. However, it can be seen that sustained release is obtained over a period of at least ten hours.

**Table 8(i)**

| Time (hours) | % Ibuprofen Dissolved | | | |
|---|---|---|---|---|
| | 0M | 3M | | |
| | | 25°C (60% RH) | 30°C (60% RH) | 40°C (75% RH) |
| 2 | 7.1 | 6.5 | 6.9 | 7.5 |
| 4 | 12.9 | 12.5 | 12.7 | 13.8 |
| 6 | 19.5 | 18.9 | 19.1 | 21.6 |
| 8 | 26.8 | 26.2 | 26.6 | 30.8 |
| 10 | 34.8 | 34.1 | 34.4 | 40.6 |
| 12 | 43.6 | 42.8 | 43.1 | 51.5 |
| 14 | 52.6 | 51.3 | 51.9 | 62.9 |
| 16 | 62.1 | 60.6 | 61.8 | 74.7 |
| 18 | 71.3 | 69.8 | 70.2 | 86.5 |
| 20 | 81.1 | 80.3 | 78.2 | 96.2 |
| 22 | 90.1 | 90.0 | 85.1 | 102.0 |

**Table 8(ii)**

| Time (hours) | % Ibuprofen Dissolved | | | | | | |
|---|---|---|---|---|---|---|---|
| | 6M | | | 9M | | 12M | |
| | 25°C (60% RH) | 30°C (60% RH) | 40°C (75% RH) | 25°C (60% RH) | 30°C (60% RH) | 25°C (60% RH) | 30°C (60% RH) |
| 2 | 6.8 | 6.7 | 11.5 | 6.6 | 6.8 | 6.8 | 6.6 |
| 4 | 12.7 | 12.7 | 30.1 | 12.8 | 12.6 | 12.5 | 12.7 |
| 6 | 19.6 | 19.6 | 53.0 | 19.9 | 19.4 | 18.9 | 20.3 |
| 8 | 27.8 | 27.2 | 78.0 | 28.1 | 27.2 | 26.1 | 29.1 |
| 10 | 35.9 | 35.4 | 96.0 | 36.4 | 35.4 | 34.0 | 38.5 |
| 12 | 45.0 | 44.3 | 103.0 | 45.8 | 44.3 | 42.6 | 49.6 |
| 14 | 53.8 | 53.2 | 103.3 | 55.1 | 53.7 | 51.1 | 60.2 |
| 16 | 63.3 | 63.5 | 103.4 | 64.6 | 63.4 | 60.6 | 71.1 |
| 18 | 72.5 | 72.6 | 103.7 | 74.5 | 73.1 | 69.1 | 80.9 |
| 20 | 82.0 | 82.1 | 103.6 | 84.0 | 82.7 | 78.5 | 90.3 |
| 22 | 90.3 | 91.2 | 103.9 | 94.2 | 92.7 | 84.2 | 98.2 |

**Table 9(i)**

| Time (hours) | % Ibuprofen Dissolved | | | |
|---|---|---|---|---|
| | 0M | 3M | | |
| | | 25°C (60% RH) | 30°C (60% RH) | .40°C (75% RH) |
| 2 | 7.9 | 6.9 | 6.2 | 7.3 |
| 4 | 13.4 | 12.9 | 12.1 | 13.9 |
| 6 | 20.0 | 19.4 | 18.3 | 21.7 |
| 8 | 28.2 | 26.9 | 26.1 | 31.2 |
| 10 | 36.8 | 34.7 | 33.1 | 40.1 |
| 12 | 46.3 | 43.1 | 42.3 | 50.5 |
| 14 | 55.3 | 51.6 | 50.2 | 59.8 |
| 16 | 65.4 | 60.8 | 60.0 | 71.8 |
| 18 | 74.8 | 69.7 | 67.9 | 82.4 |
| 20 | 84.1 | 79.6 | 75.7 | 93.0 |
| 22 | 94.2 | 88.5 | 82.8 | 99.3 |

**Table 9(ii)**

| Time (hours) | % Ibuprofen Dissolved | | | | | | |
|---|---|---|---|---|---|---|---|
| | 6M | | | 9M | | 12M | |
| | 25°C (60% RH) | 30°C (60% RH) | 40°C (75% RH) | 25°C (60% RH) | 30°C (60% RH) | 25°C (60% RH) | 30°C (60% RH) |
| 2 | 7.8 | 7.0 | 11.4 | 7.2 | 7.1 | 7.7 | 6.3 |
| 4 | 13.9 | 13.7 | 28.6 | 13.0 | 13.0 | 12.6 | 12.4 |
| 6 | 20.8 | 20.8 | 50.5 | 19.6 | 19.7 | 18.7 | 19.2 |
| 8 | 29.0 | 29.0 | 73.0 | 26.8 | 27.4 | 26.6 | 27.5 |
| 10 | 37.8 | 37.7 | 94.0 | 34.6 | 35.7 | 33.2 | 36.6 |
| 12 | 47.5 | 47.4 | 103.7 | 43.3 | 44.7 | 41.9 | 46.7 |
| 14 | 39.3 | 57.2 | 103.5 | 52.0 | 54.0 | 50.0 | 56.3 |
| 16 | 67.7 | 67.7 | 103.8 | 61.1 | 63.4 | 59.0 | 66.3 |
| 18 | 77.9 | 77.9 | 104.2 | 70.1 | 72.2 | 67.5 | 76.1 |
| 20 | 87.8 | 87.9 | 104.0 | 78.8 | 81.6 | 76.2 | 85.9 |
| 22 | 96.3 | 97.2 | 104.9 | 87.2 | 91.1 | 84.3 | 95.2 |

**Table 10(i)**

| Time (hours) | % Ibuprofen Dissolved | | | |
|---|---|---|---|---|
| | 0M | 3M | | |
| | | 25°C (60% RH) | 30°C (60% RH) | .40°C (75% RH) |
| 2 | 6.1 | 6.0 | 6.4 | 5.7 |
| 4 | 12.5 | 12.1 | 12.6 | 12.3 |
| 6 | 19.5 | 18.4 | 19.7 | 19.6 |
| 8 | 27.7 | 25.7 | 28.0 | 28.4 |
| 10 | 36.4 | 33.6 | 36.8 | 37.5 |
| 12 | 46.3 | 42.0 | 46.6 | 48.8 |
| 14 | 55.8 | 50.3 | 56.1 | 59.7 |
| 16 | 65.8 | 59.3 | 66.3 | 71.6 |
| 18 | 76.7 | 67.5 | 75.6 | 80.0 |
| 20 | 87.8 | 76.3 | 84.9 | 88.1 |
| 22 | 98.2 | 86.2 | 93.8 | 93.8 |

**Table 10(ii)**

| Time (hours) | % Ibuprofen Dissolved | | | | | | |
|---|---|---|---|---|---|---|---|
| | 6M | | | 9M | | 12M | |
| | 25°C (60% RH) | 30°C (60% RH) | 40°C (75% RH) | 25°C (60% RH) | 30°C (60% RH) | 25°C (60% RH) | 30°C (60% RH) |
| 2 | 6.6 | 7.0 | 10.2 | 6.3 | 6.5 | 6.0 | 6.0 |
| 4 | 12.3 | 12.8 | 26.6 | 11.7 | 12.2 | 11.8 | 12.0 |
| 6 | 18.6 | 19.2 | 49.3 | 17.8 | 19.2 | 18.3 | 19.2 |
| 8 | 25.6 | 26.3 | 72.8 | 24.8 | 27.9 | 26.1 | 27.9 |
| 10 | 33.1 | 34.1 | 93.0 | 32.8 | 36.5 | 34.2 | 37.0 |
| 12 | 41.8 | 41.7 | 103.0 | 41.5 | 46.4 | 43.2 | 47.0 |
| 14 | 50.5 | 49.8 | 103.9 | 50.7 | 56.4 | 52.8 | 56.4 |
| 16 | 59.6 | 57.8 | 103.9 | 60.8 | 66.5 | 63.5 | 66.6 |
| 18 | 68.5 | 66.1 | 104.5 | 70.2 | 77.0 | 73.4 | 75.4 |
| 20 | 77.5 | 74.9 | 104.2 | 79.9 | 87.8 | 83.9 | 84.4 |
| 22 | 86.2 | 83.6 | 104.3 | 89.1 | 93.6 | 91.4 | 92.4 |

### Comparison with a Standard Grade of Keltrol

The results in these tables can be contrasted with results obtained on storage for the standard grade of xanthan gum (Keltrol standard: transmittance <5%). Three different batches of standard Keltrol were taken and six tablets prepared (as described in Example 1 above using production scale compressing machines). The mean value of the % of ibuprofen dissolved for the six tablets in each of the three batches (S-U) are presented in Comparative Tables 2, 3 and 4 below. It can be seen that there is a difference between the dissolution tests carried out on tablets before storage and those after storage and there is a difference in the dissolution performance depending on the conditions of storage. There is also a much more marked deterioration of the product on storage at 40°C and 75% relative humidity.
Batch S: Comparative Table 2(i) and (ii)
Batch T: Comparative Table 3 (i) and (ii)
Batch U: Comparative Table 4 (i) and (ii)

| Comparative Table 2(i) | | | | |
|---|---|---|---|---|
| Time (hours) | % Ibuprofen Dissolved | | | |
| | 0M | 3M | | |
| | | 25°C (60% RH) | 30°C (60% RH) | 40°C (75% RH) |
| 2 | 9.4 | 8.6 | 9.0 | 13.7 |
| 4 | 14.9 | 16.5 | 16.9 | 33.0 |
| 6 | 22.6 | 26.0 | 27.1 | 56.8 |
| 8 | 32.1 | 37.2 | 38.9 | 80.9 |
| 10 | 42.6 | 49.3 | 51.6 | 97.5 |
| 12 | 54.7 | 62.2 | 63.6 | 103.0 |
| 14 | 66.2 | 75.0 | 75.2 | 103.1 |
| 16 | 78.4 | 88.2 | 86.4 | 103.2 |
| 18 | 90.4 | 98.0 | 96.2 | 103.5 |
| 20 | 98.7 | 104.7 | 103.3 | 103.8 |
| 22 | 103.4 | 105.1 | 104.5 | 103.9 |

| Comparative Table 2(ii) | | | | | | | |
|---|---|---|---|---|---|---|---|
| Time (hours) | % Ibuprofen Dissolved | | | | | | |
| | 6M | | | 9M | | 12M | |
| | 25°C (60% RH) | 30°C (60% RH) | 40°C (75% RH) | 25°C (60% RH) | 30°C (60% RH) | 25°C (60% RH) | 30°C (60% RH) |
| 2 | 8.4 | 8.6 | 31.5 | 7.1 | 7.8 | 8.0 | 8.9 |
| 4 | 15.3 | 17.1 | 72.0 | 16.0 | 15.8 | 15.7 | 18.0 |
| 6 | 24.3 | 28.0 | 98.8 | 26.4 | 25.8 | 25.3 | 30.6 |
| 8 | 35.5 | 39.6 | 102.6 | 38.5 | 39.3 | 36.8 | 45.6 |
| 10 | 48.4 | 52.3 | 102.6 | 50.4 | 51.4 | 49.4 | 60.8 |
| 12 | 60.9 | 65.2 | 103.1 | 64.0 | 67.1 | 62.4 | 78.3 |
| 14 | 73.3 | 77.8 | 103.0 | 77.3 | 80.1 | 75.2 | 93.1 |
| 16 | 85.0 | 90.3 | 103.6 | 90.4 | 94.6 | 88.5 | 102.5 |
| 18 | 95.8 | 99.8 | 103.6 | 97.9 | 100.4 | 98.0 | 104.6 |
| 20 | 102.7 | 103.0 | 103.6 | 100.5 | 101.9 | 102.4 | 104.9 |
| 22 | 104.4 | 104.2 | 103.6 | 100.8 | 102.2 | 102.9 | 105.2 |

| Comparative Table 3(i) | | | | |
|---|---|---|---|---|
| Time (hours) | % Ibuprofen Dissolved | | | |
| | 0M | 3M | | |
| | | 25°C (60% RH) | 30°C (60% RH) | .40°C (75% RH) |
| 2 | 7.6 | 8.1 | 8.2 | 9.6 |
| 4 | 15.4 | 15.1 | 14.8 | 19.4 |
| 6 | 23.1 | 22.8 | 22.4 | 31.4 |
| 8 | 32.2 | 31.7 | 30.8 | 45.3 |
| 10 | 41.2 | 40.7 | 39.9 | 59.7 |
| 12 | 51.5 | 50.5 | 50.4 | 75.2 |
| 14 | 61.5 | 59.5 | 60.0 | 89.4 |
| 16 | 72.4 | 69.0 | 70.3 | 101.7 |
| 18 | 82.2 | 77.9 | 80.2 | 106.00 |
| 20 | 91.4 | 87.5 | 89.80 | 106.5 |
| 22 | 99.7 | 97.0 | 101.0 | 106.90 |

| Comparative Table 3(ii) | | | | | | | |
|---|---|---|---|---|---|---|---|
| Time (hours) | % Ibuprofen Dissolved | | | | | | |
| | 6M | | | 9M | | 12M | |
| | 25°C (60% RH) | 30°C (60% RH) | 40°C (75% RH) | 25°C (60% RH) | 30°C (60% RH) | 25°C (60% RH) | 30°C (60% RH) |
| 2 | 8.3 | 7.9 | 14.9 | 7.5 | 7.8 | 6.8 | 8.1 |
| 4 | 14.9 | 14.9 | 36.3 | 14.9 | 13.7 | 14.8 | 15.4 |
| 6 | 22.2 | 22.0 | 60.3 | 22.5 | 21.5 | 22.1 | 23.5 |
| 8 | 30.1 | 30.1 | 83.9 | 31.7 | 30.8 | 30.8 | 32.6 |
| 10 | 38.3 | 38.9 | 100.9 | 40.9 | 40.7 | 39.6 | 42.2 |
| 12 | 47.3 | 48.3 | 103.4 | 50.5 | 51.6 | 49.2 | 52.7 |
| 14 | 56.3 | 57.5 | 103.7 | 60.1 | 61.9 | 59.2 | 63.0 |
| 16 | 64.7 | 67.2 | 103.8 | 69.9 | 73.1 | 69.5 | 74.6 |
| 18 | 73.4 | 76.3 | 103.4 | 79.0 | 83.4 | 79.4 | 86.1 |
| 20 | 82.1 | 86.0 | 104.3 | 88.2 | 93.8 | 89.5 | 95.9 |
| 22 | 91.2 | 95.8 | 104 | 98.0 | 102.4 | 98.4 | 101.4 |

| Comparative Table 4(i) | | | | |
|---|---|---|---|---|
| Time (hours) | % Ibuprofen Dissolved | | | |
| | 0M | 3M | | |
| | | 25°C (60% RH) | 30°C (60% RH) | .40°C (75% RH) |
| 2 | 8.4 | 8.5 | 8.8 | 17.5 |
| 4 | 15.9 | 15.7 | 16.1 | 62.1 |
| 6 | 24.5 | 25.2 | 25.9 | 93.9 |
| 8 | 35.8 | 37.2 | 39.1 | 102.2 |
| 10 | 49.0 | 50.7 | 52.7 | 102.4 |
| 12 | 63.1 | 64.7 | 67.6 | 102.4 |
| 14 | 77.1 | 79.0 | 81.7 | 102.3 |
| 16 | 89.5 | 90.5 | 95.9 | 102.7 |
| 18 | 99.2 | 98.9 | 103.2 | 102.7 |
| 20 | 102.6 | 102.4 | 104.8 | 103.0 |
| 22 | 103.6 | 103.0 | 105.2 | 103.5 |

| Comparative Table 4(ii) | | | | | | | |
|---|---|---|---|---|---|---|---|
| Time (hours) | % Ibuprofen Dissolved | | | | | | |
| | 6M | | | 9M | | 12M | |
| | 25°C (60% RH) | 30°C (60% RH) | 40°C (75% RH) | 25°C (60% RH) | 30°C (60% RH) | 25°C (60% RH) | 30°C (60% RH) |
| 2 | 9.4 | 8.6 | 76.9 | 7.7 | 8.3 | 8.9 | - |
| 4 | 15.9 | 16.5 | 100.7 | 15.3 | 16.5 | 16.3 | - |
| 6 | 24.6 | 26.7 | 101.6 | 24.5 | 27.9 | 26.4 | - |
| 8 | 35.7 | 39.7 | 102.1 | 36.2 | 43.2 | 39.5 | - |
| 10 | 47.7 | 55.1 | 102.3 | 47.7 | 59.3 | 54.4 | - |
| 12 | 61.5 | 71.0 | 102.3 | 61.5 | 79.2 | 69.8 | - |
| 14 | 74.9 | 86.2 | 102.5 | 73.9 | 92.9 | 83.4 | - |
| 16 | 87.5 | 98.8 | 102.5 | 86.5 | 100.0 | 95.4 | - |
| 18 | 97.7 | 103.8 | 102.6 | 94.9 | 100.5 | 101.9 | - |
| 20 | 103.4 | 104.0 | 102.4 | 99.4 | 100.7 | 103.5 | - |
| 22 | 104.5 | 102.0 | 100.4 | 99.2 | 100.8 | 103.7 | |

## Claims

1. A solid sustained release pharmaceutical composition intended to release a pharmacologically active ingredient slowly after ingestion within the body as the composition progresses along the gastro-intestinal tract comprising a compressed mixture of said pharmacologically active ingredient and a sustained release carrier comprising xanthan gum **characterised in that** the composition comprises 10-25% by weight sustained release carrier and that the xanthan gum is capable of dissolving in water at a concentration of 1 part by weight xanthan gum to 100 parts by weight water to form a solution having a transmittance (600nm) of greater than 50%.

2. A solid composition according to claim 1 **characterised in that** the sustained release carrier comprises 50% or more of said xanthan gum.

3. A solid composition according to either one of claims 1 and 2 wherein the sustained release carrier comprises at least 80% of said xanthan gum.

4. A solid composition according to any one of claims 1 to 3 **characterised in that** the sustained release carrier consists essentially of said xanthan gum.

5. A solid composition according to any one of claims 1 to 4 **characterised in that** the transmittance of said xanthan gum is in the range 75-100%.

6. A solid composition according to any one of the preceding claims **characterised in that** the pharmacologically active ingredient comprises an NSAID.

7. A solid composition according to any one of the preceding claims **characterised in that** the pharmacologically active ingredient comprises ibuprofen or flurbiprofen and salts or enantiomers thereof.

8. A solid composition according to any one of the preceding claims comprising 65-90% ibuprofen or S(+)-ibuprofen.

9. A solid composition according to any one of claims 1 to 7 **characterised by** comprising racemic flurbiprofen, S(+)-flurbiprofen or R(-)-flurbiprofen present to an extent of 10-50% by weight of the composition.

10. A solid composition according to any one of the preceding claims in the form of a tablet.

11. A process to prepare a solid dosage form according to any one of the preceding claims comprising mixing the sustained release carrier with the pharmacologically active ingredient and said xanthan gum and compressing the mixture to produce a solid formulation.

12. A process according to claim 11 **characterised in that** a minor proportion of said xanthan gum is included in a pre-granulated form of the active Ingredient and a major proportion of said xanthan gum is combined with the pre-granulated active ingredient and basing excipients prior to compression into the solid dosage form.

## Patentansprüche

1. Feste pharmazeutische Retardzusammensetzung zur langsamen Freisetzung eines pharmakologisch aktiven Bestandteils im Körper nach Einnahme bei der Fortbewegung der Zusammensetzung durch den Magen-Darm-Trakt, enthaltend eine verpreßte Mischung des pharmakologisch aktiven Bestandteils und einen Xanthangummi enthaltenden Retardträger, **dadurch gekennzeichnet, daß** die Zusammensetzung 10-25 Gew.-% Retardträger enthält und das Xanthangummi in Wasser in einer Konzentration von 1 Gewichtsteil Xanthangummi auf 100 Gewichtsteile Wasser löslich ist und dabei eine Lösung mit einer Durchlässigkeit (600 nm) von mehr als 50% bildet.

2. Feste Zusammensetzung nach Anspruch 1, **dadurch gekennzeichnet, daß** der Retardträger mindestens 50% des Xanthangummis enthält.

3. Feste Zusammensetzung nach Anspruch 1 oder 2, **dadurch gekennzeichnet, daß** der Retardträger mindestens 80% des Xanthangummis enthält.

4. Feste Zusammensetzung nach einem der Ansprüche 1 bis 3, **dadurch gekennzeichnet, daß** der Retardträger im wesentlichen aus dem Xanthangummi besteht.

5. Feste Zusammensetzung nach einem der Ansprüche 1 bis 4, **dadurch gekennzeichnet, daß** die Durchlässigkeit des Xanthangummis im Bereich von 75-100% liegt.

6. Feste Zusammensetzung nach einem der vorhergehenden Ansprüche, **dadurch gekennzeichnet, daß** der pharmakologisch aktive Bestandteil ein NSAID enthält.

7. Feste Zusammensetzung nach einem der vorhergehenden Ansprüche, **dadurch gekennzeichnet, daß** der pharmakologisch aktive Bestandteil Ibuprofen oder Flurbiprofen und Salze oder Enantiomere davon enthält.

8. Feste Zusammensetzung nach einem der vorhergehenden Ansprüche, enthalted 65-90% Ibuprofen oder S(+)-Ibuprofen.

9. Feste Zusammensetzung nach einem der Ansprüche 1 bis 7, **dadurch gekennzeichnet, daß** sie racemisches Flurbiprofen, S(+)-Flurbiprofen oder R(-)-Flurbiprofen in einer Menge von 10-50 Gew.-%, bezogen auf die Zusammensetzung, enthält.

10. Feste Zusammensetzung nach einem der vorhergehenden Ansprüche in Form einer Tablette.

11. Verfahren zur Herstellung einer festen Dosisform nach einem der vorhergehenden Ansprüche, bei dem man den Retardträger mit dem pharmakologisch aktiven Bestandteil und dem Xanthangummi vermischt und die Mischung zu einer festen Formulierung verpreßt.

12. Verfahren nach Anspruch 11, **dadurch gekennzeichnet, daß** man vor dem Verpressen zu der festen Dosisform einen kleineren Teil des Xanthangummis in eine vorgranulierte Form des aktiven Bestandteils einarbeitet und einen größeren Teil des Xanthangummis mit dem vorgranulierten aktiven Bestandteil und Grundträgerstoffen vereinigt.

## Revendications

1. Composition pharmaceutique solide à libération prolongée destinée à libérer lentement, après ingestion, un ingrédient pharmacologiquement actif dans le corps au fur et à mesure de la progression de la composition dans le tractus gastro-intestinal, comprenant un mélange comprimé dudit ingrédient pharmacologiquement actif et d'un excipient à libération prolongée comprenant de la gomme xanthane, **caractérisée en ce que** la composition contient de 10 à 25% en poids de l'excipient à libération prolongée et que la gomme xanthane est capable de se dissoudre dans l'eau à une concentration de 1 partie en poids de gomme xanthane pour 100 parties en poids d'eau pour former une solution présentant un facteur de transmission (600 nm) supérieur à 50%.

2. Composition solide selon la revendication 1, **caractérisée en ce que** l'excipient à libération prolongée contient 50% ou plus de ladite gomme xanthane.

3. Composition solide selon l'une des revendications 1 et 2, où l'excipient à libération prolongée contient au moins 80% de ladite gomme xanthane.

4. Composition solide selon l'une quelconque des revendications 1 à 3, **caractérisée en ce que** l'excipient à libération prolongée consiste essentiellement en ladite gomme xanthane.

5. Composition solide selon l'une quelconque des revendications 1 à 4, **caractérisée en ce que** le facteur de transmission de ladite gomme xanthane est de l'ordre de 75 à 100%.

6. Composition solide selon l'une quelconque des revendications qui précèdent, **caractérisée en ce que** l'ingrédient pharmacologiquement actif contient un AINS (anti-inflammatoire non stéroïdien).

7. Composition solide selon l'une quelconque des revendications qui précèdent, **caractérisée en ce que** l'ingrédient pharmacologiquement actif contient de l'ibuprofen ou du flurbiprofen et des sels ou des énantiomères de ceux-ci.

8. Composition solide selon l'une quelconque des revendications qui précèdent, contenant de 65 à 90% d'ibuprofen ou de S(+)-ibuprofen.

9. Composition solide selon l'une quelconque des revendications 1 à 7, **caractérisée en ce qu'**elle contient du flurbiprofen racémique, du S(+)-flurbiprofen ou du R(-)-flurbiprofen, en quantité de 10 à 50% en poids de la composition.

10. Composition solide selon l'une quelconque des revendications qui précèdent, sous forme de comprimé.

11. Procédé de préparation d'une forme de dosage solide selon l'une quelconque des revendications qui précèdent, comprenant le mélange de l'excipient à libération prolongée avec l'ingrédient pharmacologiquement actif et ladite gomme xanthane, et la compression du mélange pour produire une formulation solide.

12. Procédé selon la revendication 11, **caractérisé en ce qu'**une proportion mineure de ladite gomme xanthane est introduite dans une forme pré-granulée de l'ingrédient pharmacologiquement actif et qu'une proportion majeure de ladite gomme xanthane est combinée à l'ingrédient actif pré-granulé et à des excipients de base avant la compression en la forme de dosage solide.
